# EUROPEAN PATENT APPLICATION

(11) **EP 3 903 666 A1**
(43) Date of publication of application: **03.11.2021**
(21) Application number: 19905160.8
(22) Date of filing: 24.12.2019
(51) Int. Cl.: A61B 5/00, G16H 50/20, G16H 80/00, G01N 33/49, G01N 33/493, G01N 33/74

(54) **DISEASE PREDICTING SYSTEM**

(30) Priority: 25.12.2018 JP 2018241064
(71) Applicant: Kyocera Corporation, Kyoto-shi, Kyoto 612-8501 (JP)
(72) Inventor: WATANABE,Kenichi, Kyoto-shi, Kyoto 612-8501 (JP); KYOMOTO,Masayuki, Kyoto-shi, Kyoto 612-8501 (JP)
(74) Representative: Viering, Jentschura & Partner mbB Patent- und Rechtsanwälte
(86) International application number: PCT/JP2019/050611
(87) International publication number: WO 2020/138085

(57) **Abstract**

A disease predicting system according to an embodiment of the present invention includes an input unit into which input information including first information of a subject used in diagnosis of a disease and second information relating to a hormone-like agent of the subject is input; and a control unit configured to predict a future onset of the disease for the subject from the input information input into the input unit.

## Description

### Technical Field

The present disclosure relates to a disease predicting system.

### Background Art

In Patent Document 1, a diagnosis assisting apparatus for the disease osteoporosis is described.

### Citation List

### Patent Literature

Patent Document 1: Japanese application publication No. 2008-36068

### Summary

There is a demand for predicting the future onset of diseases in order to know the risk of being beset with the diseases in the future.

A disease predicting system according to an embodiment of the present invention includes an input unit into which input information including first information of a subject used in diagnosis of a disease and second information relating to a hormone-like agent of the subject is input; and a control unit configured to predict a future onset of the disease for the subject from the input information input into the input unit.

### Brief Description of Drawings

FIG. 1 is a conceptual diagram schematically illustrating the configuration of a disease predicting system 1 of the present disclosure.
FIG. 2 is a conceptual diagram schematically illustrating the configuration of a portion of the disease predicting system 1 of the present disclosure.
FIG. 3 is a conceptual diagram schematically illustrating the configuration of a portion of the disease predicting system 1 of the present disclosure.
FIG. 4 is a conceptual diagram schematically illustrating the configuration of a portion of the disease predicting system 1 of the present disclosure.
FIG. 5 is a conceptual diagram schematically illustrating the configuration of a portion of the disease predicting system 1 of the present disclosure.

### Description of Embodiments

A disease predicting system 1 of the present disclosure is capable of predicting the onset of a disease in middle to advance aged persons, for example. Diseases associated with middle to advance aged persons include, for example, osteoporosis, osteoarthritis, spondylosis, bone fractures, sarcopenia, frailty, menopausal disorders, erectile dysfunction (ED), and periodontal diseases.

Note that in the following examples, if not specifically stated, the disease in question is osteoporosis.

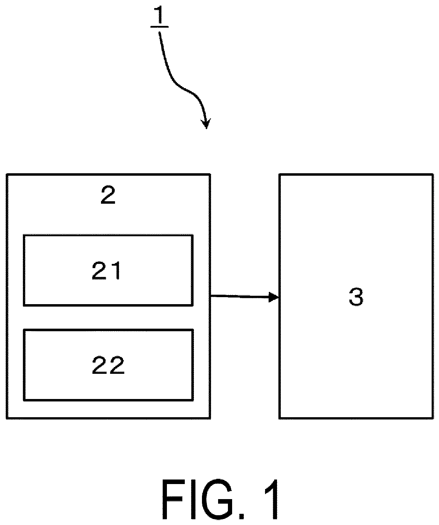

FIG. 1 is a conceptual diagram of the configuration of the disease predicting system 1 of the present disclosure.

The disease predicting system 1 of the present disclosure includes a terminal apparatus 2 and a prediction apparatus 3. The terminal apparatus 2 is configured to acquire subject data used in diagnosis of a disease. The prediction apparatus 3 is capable of predicting the future onset of a disease on the basis of the data acquired by the terminal apparatus 2.

As described above, the terminal apparatus 2 is capable of acquiring the data. The data acquired by the terminal apparatus 2 is input to the prediction apparatus 3 as input information I. Note that the input information I includes information relating to the hormone-like agent of the subject (second information 12) in addition to the subject data (first information I1) used in diagnosis of the disease. The terminal apparatus 2 includes a first terminal apparatus 21 that acquires the first information I1 and a second terminal apparatus 22 that acquires the second information.

In a case where, for example, the disease predicting system 1 is used to diagnose osteoporosis, the first terminal apparatus 21 is, for example, a simple x-ray imaging device or a bone mass measuring device. In addition, in this case, the first information I1 may include a medical image such as a simple x-ray image, for example.

The second terminal apparatus 22 is capable of acquiring the second information 12 affecting a disease associated with the first information I1 acquired by the first terminal apparatus 21. The second information 12 is information of the presence or absence or concentration of a hormone-like agent, for example. The second information 12 is a measurement value of a hormone-like agent in the blood or urine of the subject, for example. Specifically, the second information 12 is data indicating the presence or absence of non-steroidal estrogens in a case where the first information I1 relates to osteoporosis. Note that the second terminal apparatus 22 is an inspection device using a surface acoustic wave (SAW) sensor, for example.

After acquiring the input information I, the terminal apparatus 2 may transfer the input information I to the prediction apparatus 3. For example, in a case where a simple x-ray image of a subject is acquired, the terminal apparatus 2 (the first terminal apparatus 21) is installed, for example, in an x-ray room, and takes an x-ray image of the subject. The image data is transferred from the terminal apparatus 2 to the prediction apparatus 3, and the future onset of the disease can be predicted via the prediction apparatus 3.

Note that the terminal apparatus 2 does not need to directly transfer the input information I to the prediction apparatus 3. In this case, for example, the input information I acquired by the terminal apparatus 2 may be stored in a storage medium, and the input information I may be input to the prediction apparatus 3 via the storage medium.

After acquiring the second input information 12, the second terminal apparatus 22 may transfer the second input information 12 to the prediction apparatus 3. For example, in a case where the inspection device using a SAW sensor transfers information relating to the concentration of hormone-like agent in the blood or urine of the subject, the information may be transferred as a concentration value, or the information of the primary data before conversion to the concentration may be transferred. The primary data is information relating to phase changes, amplitude changes, frequency changes, and the like of the detection signal obtained by the sensor, for example.

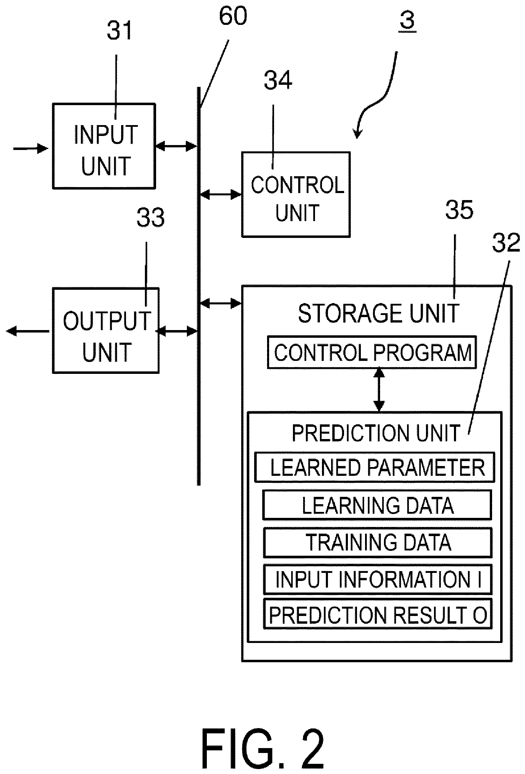

FIG. 2 is a conceptual diagram of the configuration of the prediction apparatus 3 according to the present embodiment.

The prediction apparatus 3 is capable of predicting a future onset of a disease for the subject from the input information I input to the prediction apparatus 3. For example, in a case where the disease predicting system 1 predicts the future onset of osteoporosis, the prediction apparatus 3 may predict the future onset of osteoporosis for the subject from the input information I including the medical image acquired by the terminal apparatus 2 and output a predicted prediction result O.

The prediction apparatus 3 includes an input unit 31, a control unit 34, an output unit 33, and a storage unit 35. The input unit 31, the output unit 33, the control unit 34, and the storage unit 35 are electrically connected to one another by a bus 60, for example. The input unit 31 is configured to receive the input information I from the terminal apparatus 2. The control unit 34 is capable of predicting the onset of a disease on the basis of the input information I using a prediction unit 32 described below by executing a control program. The output unit 33 is capable of outputting the prediction result O predicted by the prediction unit 32. The storage unit 35 stores a control program and various data, parameters, and the like necessary for control.

The prediction apparatus 3 according to the present disclosure includes a plurality of electronic components and circuits. In other words, various electronic components and circuits may constitute the various constituent members that form the prediction apparatus 3. The plurality of electronic components include, for example, an active element, such as a transistor or a diode, or a passive element, such as a capacitor, and is formed by a conventionally known method.

As described above, the input information I is input to the input unit 31. The input unit 31 may include a communication unit so that the input information I acquired by the terminal apparatus 2 is input directly from the terminal apparatus 2. The input unit 31 may include an input device into which the input information I or other information can be input. The input device is a keyboard, touch panel, mouse, or the like, for example.

The control unit 34 is capable of comprehensively managing the operation of the prediction apparatus 3 by controlling the other components of the prediction apparatus 3. The control unit 34 is also referred to as a control apparatus or a control circuit. The control unit 34 includes at least one processor to provide control and processing capabilities for performing various functions, as will be described in further detail below.

In one embodiment, the processor includes one or more circuits or units configured to execute one or more data computational procedures or processes by executing instructions stored in the associated memory, for example. In other embodiments, the processor may be firmware (for example, discrete logic components) configured to execute one or more data computational procedures or processes.

According to various embodiments, the processor may include one or more processors, a controller, a microprocessor, a microcontroller, an application specific integrated circuit (ASIC), a digital signal processing apparatus, a programmable logic device, a field-programmable gate array, or any combination of these devices or configurations, or any combination of other known devices and configurations, and execute the functions described below. In the present example, the control unit 34 includes a central processing unit (CPU), for example.

The storage unit 35 includes a non-transitory recording medium readable by the CPU of the control unit 34, such as read only memory (ROM) or random access memory (RAM). A control program for controlling the prediction apparatus 3 is stored in the storage unit 35. Various functions of the control unit 34 are implemented by the CPU of the control unit 34 executing the control program in the storage unit 35. The control program may also be referred to as a prediction program for causing a computer device 1 to function as the prediction apparatus 3.

In the present example, the control unit 34 executes the control program in the storage unit 35 to implement, in the control unit 34, the prediction unit 32 that is capable of estimating the prediction result O. The prediction unit 32 includes, for example, a neural network. The control program may also be referred to as a program for causing the computer device 1 to function as a neural network (the prediction unit 32). A configuration example of the neural network will be described in detail below.

In addition to the control program, the storage unit 35 stores learned parameters, estimation data (hereinafter, also referred to as "input information"), learning data, and training data relating to the neural network. The learning data and the training data are data used in training the neural network. The learned parameters and the estimation data are data used when the trained neural network estimates the onset of the disease.

The prediction unit 32 is capable of predicting the future onset of the disease for the subject from the input information I input to the input unit 31. The prediction unit 32 includes an artificial intelligence (AI). The AI of the present disclosure is, for example, a neural network.

Furthermore, the prediction unit 32 has been trained in advance. In other words, by applying machine learning to the prediction unit 32 using the learning data and the training data, the prediction unit 32 is capable of calculating the prediction result O from the input information I. Note that the learning data and the training data are data corresponding to the input information I input to the prediction apparatus 3 and the prediction result O output from the prediction apparatus 3.

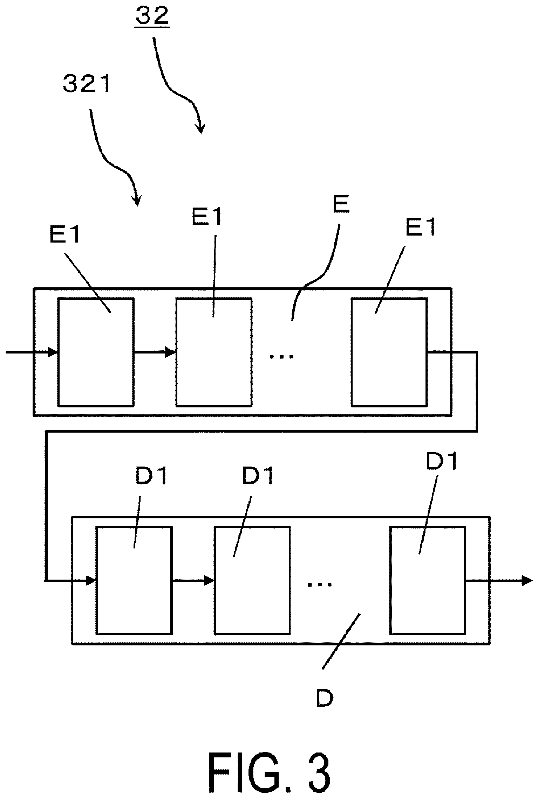

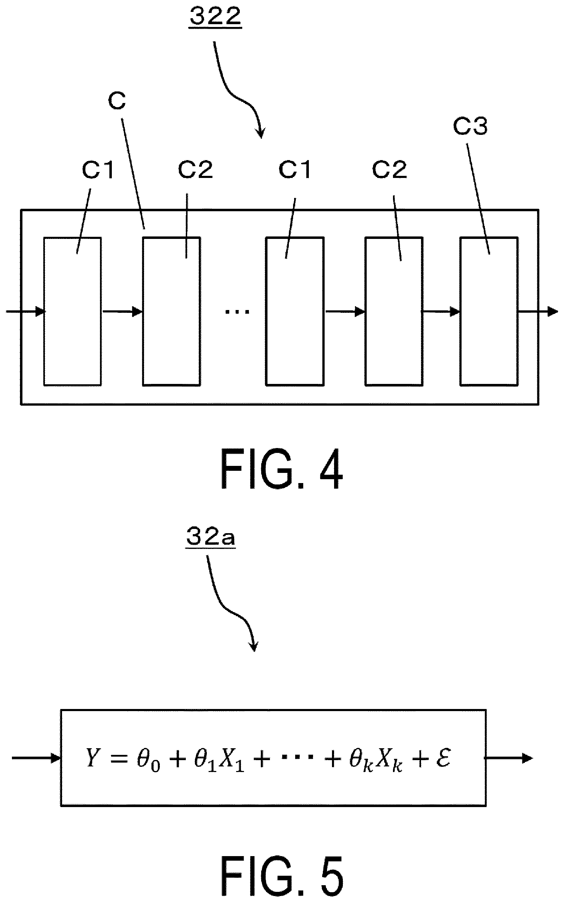

FIGS. 3 and 4 are conceptual diagrams of the configuration of the prediction unit 32 of the present disclosure.

The prediction unit 32 includes a first neural network 321 and a second neural network 322. The first neural network 321 is a neural network suitable for handling time series information. For example, the first neural network 321 is a ConvLSTM network or the like in which a convolutional neural network (CNN) and a long short-term memory (LSTM) are combined. The second neural network 322 is a convolutional network constituted by a CNN or the like, for example.

FIG. 3 is a conceptual diagram of the configuration of the first neural network 321 of the present disclosure. FIG. 4 is a conceptual diagram of the configuration of the second neural network 322.

The first neural network 321 includes an encoding unit E and a decoding unit D. The encoding unit E is capable of extracting a change in time of the input information I and a feature value of the position information. The decoding unit D is capable of calculating a new feature value on the basis of the feature value extracted by the encoding unit E, the change in time of the input information I, and the initial value.

The encoding unit E includes a plurality of convolutional long short-term memory (ConvLSTM) layers E1. The decoding unit D includes a plurality of convolutional long short-term memory (ConvLSTM) layers D1. Note that the plurality of ConvLSTM layers E1 may learn different contents. Moreover, the plurality of ConvLSTM layers D1 may learn different contents. For example, one ConvLTSM layer may learn specific contents, such as a change per 1 pixel, and another ConvLSTM layer may learn broader contents, such as an overall change.

The second neural network 322 includes a conversion unit C. The conversion unit C is capable of converting the feature value calculated by the decoding unit D into bone mass. The conversion unit C includes a plurality of convolutional layers C1, a plurality of pooling layers C2, and a fully connected layer C3. The fully connected layer C3 is positioned at the stage before the output unit 33, and the plurality of convolutional layers C1 and the plurality of pooling layers C2 are alternately arranged.

Note that at the time of training of the prediction unit 32, the learning data is input into the encoding unit E of the prediction unit 32, and the training data is compared with the output data output from the conversion unit C of the prediction unit 32.

The output unit 33 is capable of displaying the prediction result O. The output unit 33 is, for example, a liquid crystal display or an organic EL display. The output unit 33 is capable of displaying various types of information, such as characters, symbols, and graphics. The output unit 33 may display, for example, numbers, images, or the like.

### Examples of Input Information, Learning Data, and Training Data

The input information I includes first information I1 of the subject used in the diagnosis of the disease. The first information I1 is a test result or a medical image of the subject, for example. Specifically, in a case where the disease predicting system 1 is configured to predict osteoporosis, for example, the bone mass, the bone metabolism marker, an x-ray image or CT image of the chest, waist, or proximal femur, and the like of the subject are used.

Note that in the case of osteoporosis, the first information I1 specifically includes image data of a simple x-ray image showing the bone of the subject. The bone to be imaged is mainly a cortical bone or a cancellous bone of the organism, but the target bone may include an artificial bone mainly composed of calcium phosphate or a regenerated bone artificially produced by regenerative medicine or the like.

The input information I may include the second information relating to a hormone-like agent that affects the disease associated with the first information I1. The second information 12 is information of the presence or absence or concentration of a hormone-like agent, for example. In a case where the first information I1 is data relating to osteoporosis, for example, the second information 12 is information relating to a hormone-like agent that affects osteoporosis. Specifically, the second information 12 is concentration information of at least one type of fibroblast growth factor-23 (FGF23), leptin, insulin, sclerotin, or the like. Further, in a case where the subject is a female, the second information 12 is concentration information of at least one type of a steroidal estrogen, such as estrone, estradiol, and estriol, non-steroidal estrogen, progesterone, an estrogen receptor, or the like. In a case where the subject is a male, the second information 12 is concentration information of at least one type of an androgen, such as testosterone and dihydrotestosterone, an androgen receptor, or the like.

The second information 12 is information relating to a hormone-like agent that affects sarcopenia in a case where the first information I1 is data relating to sarcopenia, for example. Specifically, the second information 12 is concentration information of at least one type of ghrelin, leptin, adiponectin, or the like. In addition, in a case where the subject is a female, the second information 12 is concentration information of at least one type of a steroidal estrogen, such as estrone, estradiol, and estriol, non-steroidal estrogen, progesterone, an estrogen receptor, or the like. In a case where the subject is a male, the second information 12 is concentration information of at least one type of an androgen, such as testosterone and dihydrotestosterone, an androgen receptor, or the like.

Note that in a case where the disease predicting system 1 is configured to predict osteoarthritis and spondylosis, the first information I1 is an x-ray image of the affected area. In addition, in a case where prediction is performed for bone fractures, the first information I1 is bone mass, a bone metabolism marker, bone fracture history, or an x-ray image. In a case where prediction is performed for sarcopenia, the first information I1 is an x-ray image of the lower leg, a muscle mass, a grip strength, and walking speed. Further, in a case where prediction is performed for frailty, the first information I1 is information relating to grip force, walking speed, amount of activity, oral function, fatigue, and sociability. In the case where prediction is performed for menopausal disorders, the first information I1 is the presence or absence of hot flashes, perspiration, cold face and hands, shortness of breath, heart palpitations, difficulty in falling asleep and poor sleep quality, frustration, depression, headaches, dizziness, nausea, fatigue, and pain in the shoulders, lower back, and limbs. Further, in a case where prediction is performed for ED, the first information I1 is information relating to sexual intercourse success rate and satisfaction. In a case where prediction is performed for periodontal disease, the first information I1 is information, such as an x-ray image, a CT image, a periodontal pocket depth, an attachment level, and an oral hygiene state.

The learning data includes first learning information similar to the first input information I1. For example, if the first input information I1 is a simple x-ray image, the learning data also includes a simple x-ray image. Furthermore, in a case where the first input information I1 is bone mass, the learning data also includes bone mass.

As to the learning data, time changes are taken into account. That is, the learning data may be sets of data in which the same person is inspected on different time axes. The learning data may also be a group of data for a different person. Additionally, in a case where the learning data is image data, the image data may be sets of data in which the same site of the same person is imaged on different time axes. As a result, the prediction unit 32 that has been subjected to the learning process is capable of predicting the future onset of the disease.

The learning data includes second learning information similar to the second input information 12. That is, the learning data is information relating to the hormone-like agent of the subject. The second learning information may be information at the time of acquiring the first learning information or at a predetermined time period before or after acquiring the first learning information. For example, in a case where the subject is a female, the second input information 12 is concentration information of at least one type of a steroidal estrogen, such as estrone, estradiol, and estriol, non-steroidal estrogen, progesterone, an estrogen receptor, or the like.

Note that, similar to the input information I, the learning data may be acquired using the terminal apparatus 2. The first learning information is acquired using the first terminal apparatus 21 such as a simple x-ray device. The second learning information is acquired using the second terminal apparatus 22 such as a surface acoustic wave (SAW) sensor. In a case where the learning data is the concentration of non-steroidal estrogen, the learning data may be acquired using the second terminal apparatus 22 as described above.

The training data includes disease diagnostic results corresponding to the learning data. For example, in a case where prediction is performed for osteoporosis, the training data is an actual measurement value of bone mass or an osteoporosis diagnostic result corresponding to each of the plurality of learning image data. The actual measurement value of bone mass or the osteoporosis diagnostic result are evaluated at approximately the same time as when the learning image data is captured.

In the case of bone mass, the training data is measured by, for example, a dual-energy x-ray absorptiometry (DEXA) method or an ultrasound method, for example.

### Neural Network Learning Example

The prediction unit 32 is optimized by machine learning using the learning data and the training data so that the prediction result O can be calculated from the input information I. In other words, the control unit 34 calculates the prediction result O from the input information I on the basis of an approximation equation (prediction unit 32) optimized by machine learning. However, the machine learning is performed with the parameters in the prediction unit 32 being adjusted so that the difference between the training data and the pseudo prediction results calculated from the learning data input to the input unit 31 and output from the output unit 33 is reduced. As a result, the prediction unit 32 can perform calculation based on the learned parameter on the input information I to output the prediction result O.

A backpropagation method, for example, is used as the method for adjusting the parameter. Parameters include, for example, parameters used by the encoding unit E, the decoding unit D, the conversion unit C. Specifically, the parameters include weighting coefficients used in the ConvLSTM layers of the encoding unit E and the decoding unit D and the convolutional layers and the fully connected layer of the conversion unit C.

As described above, the disease predicting system 1 outputs the prediction result O based on the input information I, thereby predicting the onset of the disease.

Here, for example, a known osteoporosis diagnosis assisting apparatus determines osteoporosis using a feature value relating to the cortical bone. However, known diagnosis assisting apparatuses for osteoporosis diagnose osteoporosis at the current time and do not show the potential for future onset.

In contrast, the disease predicting system 1 according to the present invention is capable of predicting the future onset of a disease for the subject from the input information I. Accordingly, how the disease manifests/progresses after data acquisition can be predicted from the input information I at the time of data acquisition. Furthermore, the disease predicting system 1 according to the present invention also includes information (second information) relating to hormone-like agents as the input information I. For example, because the progression of the disease varies depending on the presence or absence of a hormone-like agent, the accuracy of future prediction can be improved by inputting the second information.

The prediction result O of the disease predicting system 1 is a future prediction of the time onward from the acquisition date of the input information I. For example, the disease predicting system 1 performs a prediction of the time from three months to 50 years after the acquisition date of the input information I, and more preferably from 6 months to 10 years.

The prediction result O may be output as a future numerical value. For example, in a case where the bone mass is predicted, the prediction result O is a numerical value expressed by at least one type of the young adult mean (YAM), a T-score, or a Z-score.

Note that in a case where prediction is performed for osteoarthritis or spondylosis, the prediction result O may be a Kellgren-Lawrence (K&L) grade or the like. In a case where prediction is performed for bone fracture, sarcopenia, frailty, menopausal disorders, ED, and periodontal diseases, the prediction result O may be the presence or absence of disease onset. The presence or absence of disease onset may be determined using a predetermined threshold value on the basis of the numerical value output by the prediction result.

The output prediction result O may be the probability of a future onset of a disease. The output onset probability may be, for example, the probability of onset at a specific date. In this case, the output is, for example, "10% probability of disease one year from now". The output may be "the time of disease onset is..." or the like. In this case, the output is, for example, "there is a possibility of you having an onset of osteoporosis seven years from now".

The output prediction result O may be a change over time. For example, the output may be a change in the prediction result O on the time axis, such as the disease onset probability one year from now, the disease onset probability five years from now, the disease onset probability ten years from now, and the like.

The disease predicting system 1 may output the prediction result O for the onset of a plurality of diseases using one type of the first information I1. In this case, for example, by inputting the concentration of non-steroidal estrogen in urine as the first information I1, the onset of the diseases of osteoporosis and menopausal disorder can be simultaneously predicted.

The disease predicting system 1 may output the prediction result O for predicting the onset of a plurality of diseases using one type of the first information I1 and one type of second information 12. In this case, for example, by inputting an x-ray image as one type of the first information I1 and the concentration of non-steroidal estrogen in urine as one type of the second information 12, for example, the onset of a plurality of diseases, such as osteoporosis and osteoarthritis, can be simultaneously predicted.

The input information I may include third information 13 including personal data of the subject. The third information 13 is information relating to the health status of the subject. Note that the personal data includes, for example, one or more types of information relating to age, gender, height, weight, systolic blood pressure, total cholesterol, neutral fat, bad cholesterol (LDL-C, neutral fat), good cholesterol (HDL-cholesterol), insulin resistance index (HOMA-R index), blood sugar level, menopause, and sperm count.

Note that in this case, third learning information similar to the third information 13 may be learned as the learning data. The third learning information may be information at the time of acquiring the first learning information or at a predetermined time period before or after acquiring the first learning information.

The third information 13 may including lifestyle information. Lifestyle information includes one or more types of information relating to dietary habits, drinking habits, smoking habits, and exercise habits, such as walking speed or number of steps.

The input information I may include fourth information 14 relating to intervention for the subject. The fourth information 14 is information relating to a scheduled (future) change for the lifestyle habits of the subject. The fourth information 14 includes, for example, one or more types of information relating to a change in diet, change in lifestyle, change in body weight, physical therapy, medication, or supplement to be taken.

Note that in this case, fourth learning information similar to the fourth information 14 may be learned as the learning data. The fourth learning information may be information at the time of acquiring the first learning information or at a predetermined time period before or after acquiring the first learning information.

Additionally, in a case where information relating to a supplement to be taken is input as the fourth information 14, the fourth information 14 includes one or more types of the information relating to taking calcium, vitamin D, vitamin K, branched-chain amino acids, flavonoids, or probiotics. Probiotics are, for example, bacillus subtilis C-3102 or the like.

In the disease predicting system 1, the output prediction result O may be a first result based on the input information I selected from the input information I excluding the fourth information 14 and a second result based on the input information I including the fourth information 14 and at least the first input information I1.

In the disease predicting system 1, as well as the prediction result O, which is a future prediction, a current diagnostic result may also be output. As a result, changes in the disease over time can be compared.

The input information I may include fifth input information 15 including bone metabolism marker information of the subject. The bone metabolism information is, for example, bone resorption or bone forming capability. Measurement can be performed using at least one type of, for example, bone resorption markers such as cross-linked N-telopeptide of type I collagen (NTX), cross-linked C-telopeptide of type I collagen (CTX), tartrate-resistant acid phosphatase (TRACP-5 b), and deoxypyridinoline (DPD); bone forming markers such as bone alkaline phosphatase (BAP) and cross-linked N-propeptide of type 1 collagen (P1NP); or bone markers such as undercarboxylated osteocalcin (ucOC). The bone resorption marker may be measured with serum or urine as a sample.

Note that in this case, fifth learning information similar to the fifth information 15 may be learned as the learning data. The fifth learning information may be information at the time of acquiring the first learning information or at a predetermined time period before or after acquiring the first learning information.

The disease predicting system 1 may include a third terminal apparatus that acquires the fifth input information 15. The second terminal apparatus 22 may also be used to simultaneously measure the second input information 12 and the fifth input information 15. For example, the DPD of the bone metabolism marker and the concentration of the non-steroidal estrogen of the hormone-like agent may be simultaneously measured from a single urine sample from the subject. In addition, the second terminal apparatus 22 may simultaneously measure a plurality of pieces of information from among a bone metabolism marker, which is one type of first input information I1, and a hormone-like agent associated with the second input information 12. Specifically, the DPD and CTX of the bone metabolism marker and the concentrations of steroidal estrogen and non-steroidal estrogen of the hormone-like agent may be simultaneously measured from a single urine sample from the subject.

### Other Embodiments

FIG. 5 is a conceptual diagram of the configuration of a prediction unit 32a of a linear regression model of the present disclosure.

In the embodiment described above, a neural network is used as the prediction unit 32. However, in other embodiments, the prediction unit 32 may be a linear regression model in which the input information is an explanatory variable, the prediction parameter is a coefficient of the explanatory variable, and the prediction result is the target variable. The prediction parameter may be optimized by the method of least squares using the learning data and the training data. That is, with the learning data as an explanatory variable vector and the training data as a target variable vector, the explanatory variable coefficient vector may be determined so that the sum of squares of the errors is minimal.

The prediction unit 32a using the linear regression model calculates a prediction result Y by inputting the input information into the explanatory variables X1, X2, ... Xk. Explanatory variable coefficients θ1, θ2, ... θk, i.e., prediction parameters, are optimized in advance by the method of least squares using the learning data and the training data.

With the prediction unit 32a using the linear regression model, a disease such as, for example, osteoporosis, bone fracture, sarcopenia, frailty, menopausal disorders, erectile dysfunction (ED), or periodontal diseases can be simply predicted.

The present invention is not intended to be limited to the examples described in the foregoing embodiments, and many variations are included as long as no contradiction arises. Furthermore, the embodiments according to the present invention can be combined as appropriate.

For example, in the example described above, a ConvLSTM network is used in cases where a neural network is used as the prediction unit 32, but the present invention is not limited to this example. For example, the prediction unit 32 may use a recurrent neural network (RNN) or a generative adversarial network (GAN). In addition, the prediction unit 32 may combine multiple neural networks. Specifically, the neural network may be a combined neural network in which a ConvLSTM network and a convolutional neural network are combined.

Furthermore, the examples described above include an example in which the prediction unit 32 includes a neural network and an example in which the prediction unit 32 includes a linear regression model. However, in the present invention, the prediction apparatus 3 of the disease predicting system 1 may have a plurality of different prediction units. For example, the prediction unit 32a using the linear regression model and the prediction 32b using a ConvLSTM neural network may be simultaneously provided.

In this case, in the disease predicting system 1, the prediction unit 32a outputs a first prediction result O1. Furthermore, the prediction unit 32b outputs a second prediction result as a second prediction result O2. As a result, for the prediction result O, the first prediction result and the second prediction result can be compared.

The disease predicting system 1 may output, as the prediction result O, a third prediction result based on the first prediction result and the second prediction result. As a result, for example, the result (third prediction result) obtained by correcting the first prediction result on the basis of the second prediction result can be used as the prediction result O.

### Reference Signs List

- 1: Disease predicting system
- 2: Terminal apparatus
- 21: First terminal apparatus
- 22: Second terminal apparatus
- 3: Prediction apparatus
- 31: Input unit
- 32: Prediction unit
- 32a: Prediction unit
- 32b: Prediction unit
- 33: Output unit
- 34: Control unit
- 35: Storage unit
- O: Prediction result
- I: Input information

## Claims

1. A disease predicting system comprising:
an input unit into which input information comprising first information of a subject used in diagnosis of a disease and second information relating to a hormone-like agent of the subject is input; and
a control unit configured to predict a future onset of the disease for the subject from the input information.

2. The disease predicting system according to claim 1, further comprising
an output unit configured to output a prediction result predicted by the control unit.

3. The disease predicting system according to claim 1 or 2, wherein
two or more of the diseases are simultaneously predicted.

4. The disease predicting system according to any one of claims 1 to 3, wherein
the second information comprises a measurement value of a hormone-like agent in blood or in urine of the subject.

5. The disease predicting system according to any one of claims 1 to 4, wherein
the prediction is a change over time.

6. The disease predicting system according to any one of claims 1 to 5, wherein
the input information comprises third information relating to a health status of the subject.

7. The disease predicting system according to any one of claims 1 to 6, wherein
the input information comprises fourth information relating to intervention for the subject.

8. The disease predicting system according to any one of claims 1 to 7, wherein
the control unit is configured to predict a first result based on the input information excluding fourth information and a second result based on the input information including the fourth information.

9. A program executable by a disease predicting system comprising a control unit, an input unit, and an output unit, the program causing the control unit to execute:
acquiring, via the input unit, input information comprising first information of a subject used in diagnosis of a disease and second information relating to a hormone-like agent of the subject;
predicting a future onset of the disease for the subject from the input information; and
outputting a prediction result predicted by the control unit.
